# EUROPEAN PATENT APPLICATION

(11) **EP 4 385 341 A1**
(43) Date of publication of application: **19.06.2024**
(21) Application number: 22855423.4
(22) Date of filing: 09.08.2022
(51) Int. Cl.: A23L 29/269, A23L 29/20, A23L 29/30, A23K 20/163

(54) **COMPOUND THICKENING AGENT AND APPLICATION THEREOF**

(30) Priority: 10.08.2021 CN 202110915854
(71) Applicant: DSM IP Assets B.V., 6411 TE Heerlen (NL); Zhejiang DSM Zhongken Biotechnology Co. Ltd, Tongxiang Jiaxing, Zhejiang 314515 (CN)
(72) Inventor: YA, Rudan, Shanghai 201203 (CN); ZHANG, Fei, Shanghai 201203 (CN); LIU, Zhoupin, Shanghai 201203 (CN); ZHAO, Jie, Shanghai 201203 (CN); YUAN, Chienkuo, Shanghai 201203 (CN)
(74) Representative: Grey, Ian Michael
(86) International application number: PCT/CN2022/111100
(87) International publication number: WO 2023/016442

(57) **Abstract**

The present invention relates to a compound thickener comprising rhamsan gum and gellan gum. In the compound thickener, rhamsan gum accounts for 50%-95% of the total weight of rhamsan gum and gellan gum. The combination of rhamsan gum and gellan gum in the compound thickener produces a synergistic effect, and its viscosity is remarkably improved. The compound thickener can be applied to products such as foods, beverages, pharmaceutical products, and personal care products, and when the compound thickener is applied as a gelling agent in soft capsule shells, the shell strength and the shell elasticity are better.

## Description

### TECHNICAL FIELD

The invention relates to a compound thickener comprising rhamsan gum and gellan gum and a preparation method thereof. The present invention also relates to use of the compound thickener in the preparation of food, beverage, pharmaceutical product, nutritional product, personal care product or pet food, and the food, beverage, pharmaceutical product, nutritional product, personal care product or pet food containing the compound thickener.

### BACKGROUND

Rhamsan gum and gellan gum are both microbial polysaccharides produced by the fermentation of *Sphingomonas,* whose main chain consists of four sugar repeating units of glucose, glucuronic acid and rhamnose at a ratio of 2:1:1. High acyl gellan gum has an L-glyceryl group attached to the 3-linked glucose residue, while rhamsan gum has a disaccharide side chain attached at the same position. The similar chemical structure enables rhamsan gum and gellan gum to have some similar properties. Both rhamsan gum and gellan gum have strong thermal stability, showing high viscosity for their solution, even at a temperature of higher than 100 °C, and further have good acid and alkali resistance and rheology properties. Gellan gum, as a hydrocolloid fermented by microorganisms, has more application potential and value in the modem food industry than gelatin and other animal-sourced hydrocolloids. In addition, gellan gum may be used as a gelling agent in a small amount, and can form a gel at an addition amount of 0. 1%-0.3%, and thus it is widely used in food or non-food fields. However, there are few reports on the application of rhamsan gum in the field of food technology.

At present, many hydrocolloids have been used as food improvers in a wide range of food categories. In addition to using a single hydrocolloid to improve food texture, multiple hydrocolloids can also be used in combination. For example, CN104721170A discloses a chewable soft capsule with mixed hydrocolloids in which gellan gum and gelatin are compounded, and the obtained matrix has synergistic effect on gelling performances. However, as the market for products is becoming increasingly diverse, and the demand for hydrocolloid properties is also becoming increasingly diverse, there is still a need to enhance or improve functionalities of products such as foodstuffs by combining hydrocolloids with different properties to make up for deficiencies of a single hydrocolloid through complementary or synergistic effects.

### DETAILED DESCRIPTION

The invention provides a compound thickener, which comprises rhamsan gum and gellan gum, wherein the rhamsan gum accounts for 50%-95% by weight of a total weight of rhamsan gum and gellan gum. In the compound thickener, rhamsan gum can account for 50%-90%, 60%-90%, 70%-90%, 80%-90%, 50%-95%, 60%-95%, 70%-95%, 75%-85%, or 80%-95% by weight of the total weight of rhamsan gum and gellan gum, preferably, rhamsan gum accounts for 60%-95% by weight of the total weight of rhamsan gum and gellan gum. In the compound thickener, rhamsan gum can account for 50%, 55%, 60%, 65%, 70%, 75%, 80%, 85%, 90% or 95% by weight of the total weight of rhamsan gum and gellan gum. In one embodiment of the present invention, rhamsan gum in the compound thickener accounts for 60%-95% by weight of the total weight of rhamsan gum and gellan gum. In one embodiment of the present invention, rhamsan gum in the compound thickener accounts for 65%-95% by weight of the total weight of rhamsan gum and gellan gum. In one embodiment of the present invention, the compound thickener is mainly composed of rhamsan gum and gellan gum compounded according to said ratios.

In the present invention, the term "thickener" refers to a substance or substances that can increase viscosity of a system, and can keep the system in a uniform and stable suspension state, or an emulsified state, or can form a gel.

Optionally, the compound thickener may further comprise other ingredients, such as one or more of dispersants and stabilizers. In the present invention, the compound thickener can further comprise a dispersant that reduces aggregation of hydrocolloids, and the dispersant can be one or more of sugars, alkali agents, alkali metal salts, organic acid salts, and inorganic acid salts, the dispersant can also be water. The compound thickener may or may not comprise other thickener ingredients, such as one or more of gum arabic, gelatin, guar gum, carrageenan, xanthan gum, pectin, locust bean gum, sodium alginate, tamarind gum, agar, chitosan, carboxymethyl cellulose, curdlan gum, tara gum and konjac gum. Preferably, the compound thickener does not comprise gelatin. The compound thickener may comprise at least 2% by weight of rhamsan gum and gellan gum. The compound thickener may comprise 80% by weight or more of rhamsan gum and gellan gum. Preferably, the compound thickener mainly consists of rhamsan gum and gellan gum as thickening ingredients. In one embodiment of the present invention, the compound thickener is a powder.

In the present invention, "a high acyl gellan gum" refers to gellan gum in the form of high acyl directly extracted from gellan gum fermentation broth without deacylation treatment, and can be used interchangeably with "a native gellan gum" in this application. In the present invention, "a low acyl gellan gum" refers to gellan gum from which acyl groups have been removed through deacylation treatment during extraction process, and can be used interchangeably with "a deacylated gellan gum" in this application. In the present invention, gellan gum having an acyl content between the high acyl gellan gum and the low acyl gellan gum is referred to as "partially deacylated gellan gum". The gellan gum in the compound thickener of the present invention can be a high acyl gellan gum, a low acyl gellan gum and/or a partially deacylated gellan gum. Preferably, the gellan gum in the compound thickener comprises partially deacylated gellan gum. In one embodiment of the present invention, the compound thickener mainly comprises rhamsan gum and a partially deacylated gellan gum.

One aspect of the present invention is to provide a method for preparing the compound thickener, comprising the steps of:
a) weighing rhamsan gum and gellan gum powder at said weight ratio;
b) mixing the weighed powder evenly; and
c) optionally, adding a dispersant.

The weighing and mixing can be performed according to conventional methods known in the art.

One aspect of the present invention is to provide a product comprising the compound thickener, wherein the product may be food, beverage, pharmaceutical product, nutritional product, personal care product or pet food. Specifically, the food may be desserts, confectioneries, dairy products, or beverages. The product can also be a delivery system for drugs or nutrients, such as soft capsules. The personal care product can be a skin care product (e.g., face mask), oral care product, or hair care product. In the product, rhamsan gum and gellan gum may be present in an amount of 0.01%-5% by weight of the total weight of the product, preferably is present in an amount of 0.02%-4% by weight of the total weight of the product, more preferably, is present in an amount of 0.3% to 2.5% by weight of the total weight of the product.

One aspect of the present invention is to provide a method for preparing a product, wherein the product may be food, beverage, pharmaceutical product, nutritional product, personal care product or pet food, and wherein the compound thickener is used during the preparation of the product. The amount of the compound thickener can be expressed as the weight percentage of rhamsan gum and gellan gum relative to the total weight of the system to be added, such as 0.01%-5% by weight, preferably 0.02%-4%, more preferably 0.3%-2.5% by weight.

One aspect of the present invention is to provide a soft capsule shell gelling agent, comprising rhamsan gum and gellan gum, wherein rhamsan gum accounts for 50%-95% by weight, such as 65%-90%, 70-90%, 75%-90%, 80%-90%, 65%-95%, 70-95%, 75%-95%, 80%-95%, or 85%-95% by weight, for example 65%, 70%, 75%, 80%, 85%, 90%, or 95% by weight of the total weight of rhamsan gum and gellan gum in the gelling agent, preferably, rhamsan gum accounts for 70%-95% by weight of the total weight of rhamsan gum and gellan gum in the gelling agent, more preferably, 75%-95% by weight of the total weight of rhamsan gum and gellan gum in the gelling agent. The gellan gum in the shell gelling agent can be a high acyl gellan gum, a low acyl gellan gum and/or a partially deacylated gellan gum, preferably, the gellan gum in the shell gelling agent comprises a partially deacylated gellan gum. In the present invention, the shell gelling agent may or may not further comprise other hydrocolloids, such as one or more of gum arabic, gelatin, guar gum, carrageenan, xanthan gum, pectin, locust bean gum, sodium alginate, tamarind gum, agar, chitosan, carboxymethyl cellulose, curdlan gum, tara gum and konjac gum, preferably the shell gelling agent does not comprise gelatin.

The present invention also provides a soft capsule shell comprising the shell gelling agent. The shell of the soft capsule further comprises a matrix, a plasticizer and water (such as deionized water). In one embodiment of the present invention, the soft capsule shell comprises 1.5%-3.5% by weight of the shell gelling agent, 20%-45% by weight of the matrix, 7%-20% by weight of the plasticizer, and 35%-55% by weight of deionized water. In one embodiment of the present invention, the shell gelling agent mainly comprises rhamsan gum and gellan gum, the matrix is dextrin, and the plasticizer is glycerin, and the soft capsule shell can be prepared by using each ingredient at said weight ratio. The dextrin is a starch decomposition product obtained by heating or acid treatment of starch, and the dextrin has no gelling properties, and can be used as a matrix and combine with the gelling agent to form a stable and robust gel network structure. The glycerin, serving as a plasticizer, can enhance flowability of the solution for the capsule shells, ensuring sufficient flowability during the preparation process of the capsule shells.

The present invention also provides a soft capsule, the shell of which comprises the shell gelling agent of the present invention.

One aspect of the present invention is to provide a method for preparing a soft capsule shell, comprising the steps of:
a) weighing the shell gelling agent of the present invention and the matrix and mixing them at a certain weight ratio, to obtain a mixture;
b) dispersing the mixture obtained in step a) into a solution of the plasticizer and deionized water, with stirring evenly, to obtain a dispersion;
c) heating and incubating the dispersion, introducing the dispersion into a mold, cooling and setting,
wherein the shell gelling agent: matrix: plasticizer: deionized water are present at a weight ratio of 1.5-3.5: 20-45: 7-20: 35-55, the shell gelling agent mainly comprises rhamsan gum and gellan gum, and the rhamsan gum accounts for 50%-95% by weight, such as 65%-90%, 70%-90%, 75%-90%, 80%-90%, 65%-95%, 70-95%, 75%-95%, 80%-95%, or 85%-95% by weight, for example 65%, 70%, 75%, 80%, 85%, 90%, or 95% by weight of the total weight of rhamsan gum and gellan gum, preferably, the rhamsan gum accounts for 70%-95% by weight of the total weight of rhamsan gum and gellan gum, more preferably, 75%-95% by weight of the total weight of rhamsan gum and gellan gum. The gellan gum in the shell gelling agent can be a high acyl gellan gum, a low acyl gellan gum and/or a partially deacylated gellan gum, preferably, the gellan gum in the shell gelling agent comprises a partially deacylated gellan gum. In one embodiment of the present invention, the soft capsule shell may be prepared by the steps of: weighing rhamsan gum, gellan gum and dextrin at said weight ratio and fully mixing to obtain a mixture; dispersing the mixture into a solution of glycerin and deionized water, with stirring evenly to obtain a matrix solution; placing the evenly stirred matrix solution in a water bath preset at about 95°C for heat preservation, and heating for 1.5-4 hours; after the heating, pouring the gel solution into a mold and cooling the gel solution in the mold to room temperature and setting overnight, wherein the weight ratio of rhamsan gum and gellan gum: dextrin: glycerol: deionized water is 1.5-3.5: 20-45: 7-20: 35-55, and rhamsan gum accounts for 70%-95% by weight of the total weight of rhamsan gum and gellan gum.

The present invention also provides use of the compound thickener as a shell gelling agent in the preparation of soft capsule shells.

Without wishing to be bound by any theory or explanation, the inventors found that by compounding rhamsan gum and gellan gum in a specific ratio, a synergistic viscosity-increasing effect can be produced. The synergistic viscosity-increasing effect is reflected in compounding rhamsan gum and gellan gum at specific ratios to prepare a solution of a certain concentration, which results in a higher solution viscosity compared to the same concentration of solutions when using rhamsan gum or gellan gum alone. The gellan gum can be a high acyl gellan gum, a low acyl gellan gum and/or a partially deacylated gellan gum, all of which can produce a synergistic viscosity-increasing effect. Preferably, the gellan gum is partially deacylated gellan gum. When rhamsan gum and gellan gum are compounded, rhamsan gum accounts for 50%-95% by weight, such as 50%-90%, 60%-90%, 70%-90%, 80%-90%, 50%-95%, 60%-95%, 70%-95%, 75%-85%, or 80%-95% by weight of the total weight of rhamsan gum and gellan gum. The solution may have a concentration of 0.01%-5% by weight, preferably 0.02%-4% by weight, more preferably 0.3%-2.5% by weight. The viscosity of the solution prepared by compounding rhamsan gum and gellan gum is higher, compared to the solutions of the same concentration using rhamsan gum or gellan gum alone, by at least about 5%, 6%, 7%, 8%, 9%, 10%, 15%, or 20% relative to the higher of the viscosity obtained by using rhamsan gum or gellan gum alone.

In the present invention, a rheometer can be used to test a solution viscosity, with the test conditions at a shear rate of 100 1/s, a test temperature of 85° C, a strain of 2%, and a frequency of 1 Hz. In the present invention, according to the aforementioned test conditions, at a solution concentration of 1.2% by weight, the high acyl gellan gum can have a viscosity of 0.4 Pa s to 1.5 Pa s, the partially deacylated gellan gum can have a viscosity of 0.05 Pa s to 0.5 Pa s, the low acyl gellan gum can have a viscosity of 0.001 Pa s to 0.08 Pa s, and the rhamsan gum can have a viscosity of 0.1 Pa·s to 0.8 Pa·s. In one embodiment of the present invention, the viscosity is measured according to the following method: compounding and mixing rhamsan gum and gellan gum in a specific ratio to obtain a mixture, putting the mixture into deionized water, and dispersing evenly to form a solution; heating the solution to 85°C , keeping the temperature for 1 minute, adding the solution to the rheometer, and starting the test in the set composite viscosity test mode, wherein the shear rate is 100 1/s, the test temperature is 85 °C, the strain is 2%, and the frequency is 1 Hz. When measuring viscosity, the total concentration of rhamsan gum and gellan gum in the solution can be 0.5%-3% by weight, preferably 1.0%-2.5% by weight.

Without wishing to be bound by any theory or explanation, the inventors found that by compounding rhamsan gum and gellan gum in a specific ratio, gelling properties of the compounded composition also have a synergistic effect. The synergistic effect is reflected in that the gel strength of the compounded composition is higher than that of using rhamsan gum alone or using gellan gum alone at the same conditions. Specifically, when the composition is used as a shell gelling agent of a soft capsule, the strength of the soft capsule shell can be enhanced. In the shell gelling agent, rhamsan gum accounts for 50%-95% by weight, such as 65%-90%, 70-90%, 75%-90%, 80%-90%, 65%-95%, 70-95%, 75%-95%, 80%-95%, or 85%-95% by weight, for example 65%, 70%, 75%, 80%, 85%, 90%, or 95% by weight of the total weight of rhamsan gum and gellan gum in the gelling agent, preferably, rhamsan gum accounts for 70%-95% by weight of the total weight of rhamsan gum and gellan gum in the gelling agent, more preferably, accounts for 75%-95% by weight of the total weight of rhamsan gum and gellan gum in the gelling agent. The gellan gum in the shell gelling agent can be a high acyl gellan gum, a low acyl gellan gum and/or a partially deacylated gellan gum, preferably, the gellan gum in the shell gelling agent comprises a partially deacylated gellan gum. When gellan gum is used alone as a gelling agent in a soft capsule shell, elasticity of the soft capsule shell is usually not good. When rhamsan gum is used alone, operation is not convenient enough since the gelling temperature is high. When the compound thickener comprising rhamsan gum and gellan gum is used as a gelling agent, the shell strength can be higher than that obtained by using rhamsan gum or gellan gum alone, and relative to the higher of the shell strengths obtained by using rhamsan gum or gellan gum alone, using the shell gelling agent of the present invention can increase the shell strength by at least about 2%, 3%, 4%, 5%, 6%, 7%, 8%, 9%, 10%, 15%, or 20%.

In the present invention, the gel strength and elasticity can be measured by a texture analyzer. In the present invention, the gel strength refers to the maximum pressure value that the gel can bear within a certain strain range per unit area. The gel elasticity refers to a ratio of the distance travelled by a texture analyzer probe from the start of the probe contacting the gel block to the measurement and obtainment of the gel strength and the distance travelled by the texture analyzer probe from the start of the probe contacting the gel block to the measurement and obtainment of a set strain, and the ratio of distances travelled by the probe may reflect elasticity. Specifically, the texture analyzer can be used for testing according to the following settings with a probe of P/1KS, a strain of 95%, a trigger force of 5g, a pre-test speed of 1mm/sec, a test speed of 1mm/sec, and a post-test speed of 10mm/sec.

The compound thickener of the present invention, by compounding rhamsan gum and gellan gum in specific ratios, can produce a synergistic viscosity-increasing effect, and can achieve a more prominent thickening effect with a smaller amount of hydrocolloids than using a single hydrocolloid alone and cost savings. In addition, the compound thickener of the present invention also has a synergistic effect on gelling performances. When used as a shell gelling agent for soft capsule shells, the resulting capsule shells have a better shell strength than that of using rhamsan gum or gellan gum alone while maintaining good elasticity.

### DESCRIPTION OF THE DRAWINGS

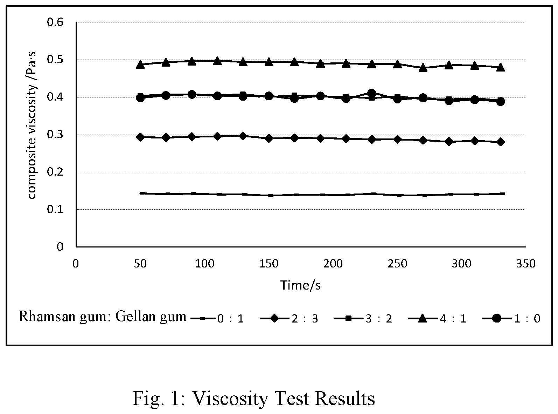

Fig. 1 shows viscosity test results. Rhamsan gum, gellan gum, and rhamsan gum and gellan gum compositions at a rhamsan gum addition ratio of 40%, 60% and 80% respectively were fully hydrated in deionized water at a concentration of 1.2%, and after heating, the resulting solutions were subjected to scanning tests on a rheometer to obtain the results of viscosity change with time.

### EXAMPLES

The present invention is further illustrated by way of examples. These descriptions are only exemplary and intended to illustrate the present invention, not to limit the protection scope of the present invention. Rhamsan gum used in the examples was purchased from Zhejiang DSM Zhongken Biotechnology Co., Ltd., and gellan gum was GELLANEER HS gellan gum purchased from Zhejiang DSM Zhongken Biotechnology Co., Ltd.

### Example 1: Viscosity increasing effect

Rhamsan gum, gellan gum, and rhamsan gum and gellan gum compositions at a rhamsan gum addition ratio of 40%, 60% and 80% respectively were fully hydrated in deionized water at a concentration of 1.2%. After heating, the scanning tests of viscosity change with time was carried out on the rheometer, and the test results were shown in Fig. 1. When the addition ratio of rhamsan gum in the composition of rhamsan gum and gellan gum was higher than 60%, the viscosity of the compounded gums was higher than that when rhamsan gum or gellan gum was used alone.

η* was the viscosity measured in the rheometer at an oscillating mode, which reflects viscoelasticity of the solution. The rheological test method was carried out at a shear rate of 100 1/s, a test temperature of 85°C, a strain of 2%, and a frequency of 1Hz.

### Example 2: Gel strength and elasticity in a capsule system

Experimental method: 2.5% of a shell gelling agent was mixed with 36.5% of maltodextrin (MD20, Baolingbao Biological Co., Ltd.) evenly, and 14% of glycerin was poured into 47% of deionized water with stirring evenly. The evenly mixed powder was added into the glycerin solution and then the mixture was stirred well. The 2.5% of a shell gelling agent in each group of experiments was: 2.5% of gellan gum; 2.5% of a gelling agent at a rhamsan gum-gellan gum ratio of 2:3; 2.5% of a gelling agent at a rhamsan gum-gellan gum ratio of 3:2; 2.5% of a gelling agent at a rhamsan gum-gellan gum ratio of 4:1; and 2.5% of rhamsan gum.

Gel strength and elasticity tests were performed with a texture analyzer (TA. XT. plus, SMS^{®}). The gel strength refers to the maximum pressure value that the gel can bear within a certain strain range per unit area. The gel elasticity refers to a ratio of the distance travelled by a texture analyzer probe from the start of the probe contacting the gel block to the measurement and obtainment of the gel strength and the distance travelled by the texture analyzer probe from the start of the probe contacting the gel block to the measurement and obtainment of a set strain, and the ratio of distances travelled by the probe may reflect elasticity. The measurement method was carried out with a probe of P/1KS at a strain of 95%, a trigger force of 5g, a pre-test speed of 1mm/sec, a test speed of 1mm/sec, and a post-test speed of 10mm/sec. The test results were shown in Table 1.

**Table 1: Test Results of Gel Strength and Elasticity**

| | 100% Gellan Gum | Rhamsan gum and Gellan Gum at a ratio of 2:3 | Rhamsan gum and Gellan Gum at a ratio of 3:2 | Rhamsan gum and Gellan Gum at a ratio of 4:1 | 100% Rhamsan gum |
|---|---|---|---|---|---|
| Gel strength (g/cm²) | 2907.9 | 3067.8 | 3267.9 | 4697.5 | 4310.7 |
| Elasticity (%) | 0.93 | 0.97 | 0.98 | 0.98 | 1.00 |

From the above results, it can be seen that the combination of rhamsan gum and gellan gum at different concentration ratios showed differential results of shell strength and elasticity in the simulated capsule shells. When the content of rhamsan gum relative to the total weight of rhamsan gum and gellan gum in the gel was 80%, the shell strength of soft capsule was significantly higher than the soft capsule shell strength of using rhamsan gum alone and the combination of these two gums produced a synergistic effect, improving the shell strength of the soft capsule, and allowing the shell to maintain ideal elasticity.

## Claims

1. A compound thickener, comprising rhamsan gum and gellan gum, wherein the rhamsan gum accounts for 50%-95% by weight of a total weight of the rhamsan gum and the gellan gum in the compound thickener.

2. The compound thickener as claimed in claim 1, wherein the rhamsan gum accounts for 60%-95% by weight of the total weight of the rhamsan gum and the gellan gum in the compound thickener.

3. The compound thickener as claimed in claim 1, wherein the rhamsan gum accounts for 65%-95% by weight of the total weight of the rhamsan gum and the gellan gum in the compound thickener.

4. The compound thickener as claimed in any one of claims 1 to 3, wherein the gellan gum comprises a high acyl gellan gum, a low acyl gellan gum and/or a partially deacylated gellan gum.

5. A product comprising the compound thickener as claimed in any one of claims 1 to 4, wherein the product is food, beverage, pharmaceutical product, nutritional product, personal care product or pet food.

6. The product as claimed in claim 5, wherein the rhamsan gum and the gellan gum are present in an amount of 0.01%-5% by weight of a total weight of the product, preferably is present in an amount of 0.02%-4% by weight of the total weight of the product.

7. A soft capsule shell gelling agent, comprising rhamsan gum and gellan gum, wherein the rhamsan gum accounts for 50%-95% by weight, preferably 70%-95% by weight, more preferably 75%-95% by weight of a total weight of the rhamsan gum and the gellan gum in the gelling agent.

8. The shell gelling agent as claimed in claim 7, wherein the gellan gum comprises a high acyl gellan gum, a partially deacylated gellan gum and/or a low acyl gellan gum.

9. A soft capsule shell comprising the shell gelling agent as claimed in claim 7 or 8, wherein the soft capsule shell further comprises a matrix, a plasticizer and water.

10. The soft capsule shell as claimed in claim 9, comprising 1.5%-3.5% by weight of the shell gelling agent, 20%-45% by weight of the matrix, 7%-20% by weight of the plasticizer, and 35%-55% by weight of water.

11. A soft capsule comprising the shell as claimed in claim 9 or 10.

12. A method for preparing the compound thickener as claimed in any one of claims 1 to 4, comprising the steps of:
a) weighing rhamsan gum and gellan gum powder at said weight ratio;
b) mixing the weighed powder evenly; and
c) optionally, adding a dispersant.

13. A method for preparing the soft capsule shell as claimed in claim 9 or 10, comprising the steps of:
a) weighing the shell gelling agent and the matrix and mixing them at a certain weight ratio, to obtain a mixture;
b) dispersing the mixture obtained in step a) into a solution of the plasticizer and water, with stirring evenly, to obtain a dispersion; and
c) heating and incubating the dispersion, introducing the dispersion into a mold, cooling and setting.

14. Use of the compound thickener as claimed in any one of claims 1 to 4 as a shell gelling agent in the preparation of soft capsule shells.
